# EUROPÄISCHE PATENTSCHRIFT

(11) **EP 0 611 578 B1**
(45) Veröffentlichungstag und Bekanntmachung des Hinweises auf die Patenterteilung: **07.01.1998**
(21) Anmeldenummer: 94100957.3
(22) Anmeldetag: 24.01.1994
(51) Int. Cl.: A61M 1/00, F04B 49/06

(54) **Medizintechnische Pumpe**
Medical pump
Pompe médicale

(30) Priorität: 19.02.1993 CH 533/93
(43) Veröffentlichungstag der Anmeldung: 24.08.1994
(73) Patentinhaber: NEOVATION AG, CH-6205 Eich (CH)
(72) Erfinder: Berner, Werner, Dr., CH-5016 Obererlinsbach (CH); Riedweg, Robert, Dr., CH-6205 Eich (CH)
(74) Vertreter: Ritscher, Thomas, Dr.

(56) Entgegenhaltungen:
- EP-A- 0 330 845
- EP-A- 0 401 067
- DE-A- 3 820 211
- DE-A- 4 031 708

## Beschreibung

Die vorliegende Erfindung betrifft eine medizintechnische Pumpe nach dem Oberbegriff des Patentanspruchs 1.

Unter medizintechnischen Pumpen sind alle Pumpen zu verstehen, welche auf dem Gebiet der technischen Medizin einsetzbar sind. Insbesondere sind darunter Pumpen zu verstehen, mit welchen besondere Sekrete abgepumpt werden, bspw. Brustpumpen für das Abpumpen der Muttermilch, oder Pumpen mit welchen ein für eine bestimmte Operation notwendiger Unter- resp. Ueberdruck gewährleistet sein muss, bspw. Thoraxpumpen bei Thoraxoperationen.

Brustpumpen sind seit längerem bekannt und werden überall dort eingesetzt, wo das direkte Stillen durch die Mutter nicht möglich oder erwünscht ist. So kann bspw. wegen der ungenügenden Saugkraft des Neugeborenen das Einsetzen der Milchproduktion nicht genügend stimuliert werden oder wegen der hohen Empfindlichkeit und Verletzbarkeit der Brustwarzen das Stillen eines saugkräftigen Babys nicht möglich sein. Physische wie psychische Beschwerden vielerlei Art können mit dem Einsatz von geeigneten Brustpumpen gemildert oder geheilt werden. Deshalb sind Brustpumpen in den verschiedensten Ausführungsformen in privaten Haushalten oder Spitälern verbreitet. So sind heute Handpumpen und elektrisch angetriebene Absauggeräte im Einsatz. Es versteht sich, dass je nach dem Grund der Stillunfähigkeit die einen oder anderen Brustpumpen vorteilhafterweise verwendet werden. So sind elektrisch angetriebene Brustpumpen bspw. überall dort angezeigt, wo der Saugrhythmus und/oder eine bestimmte Saugkraft erforderlich ist.

Eine elektrisch angetriebene Brustpumpe ist bspw. in der EP-A-330'845 beschrieben. Mit dieser Pumpe kann ein Unterdruck zwischen 100 mmHg und 250 mmHg mit einer Pumpfrequenz von 50 - 60 Zyklen pro Minute erzeugt werden. Der erzeugte Unterdruck und dessen zeitlicher Verlauf ist durch eine besonders gestalte Drehscheibe festgelegt und kann von der Benutzerin nicht verändert werden. Es ist aber in vielen Fällen erwünscht den maschinell erzeugten Unterdruck manuell begrenzen zu können.

In der DE-3820211 wird deshalb ein medizinisches Absauggerät beschrieben, bei welchem der momentane Unterdruck von einem Drucksensor erfasst wird und dessen elektrisches Signal zur Begrenzung des erzeugten Unterdrucks verwendet wird. Dazu wird vorgeschlagen, entweder den Saugtakt der Pumpe bei einem bestimmten (frei wählbaren oder fest einstellbaren) Unterdruckgrenzwert zu unterbrechen, oder das Luftabsaugvolumen einer volumenstromveränderbaren Vakuumpumpe beim Erreichen dieses bestimmten Unterdruckgrenzwertes zu begrenzen. Diese Vorrichtung lässt sich ebenfalls nicht universell einsetzen, erweist sich als mechanisch aufwendig (Servo-Antrieb für die Pumpe resp. Schaltung von zusätzlichen Ventilanordnungen) und setzt einen korrekten, d.h. passgenauen Sitz der Saugglocke voraus.

Bei der operativen Behandlung eines Pneumothorax wird in den durch die verletzte Pleura entstandenen Hohlraum zwischen Brustkorb und kolabierten Lungenflügel ein Unterdruck erzeugt, um diesen Lungenflügel wieder aufzuspannen. Dabei dringt in aller Regel sowohl lungenseitig als auch brustkorbseitig mehr oder weniger viel Luft in diesen Hohlraum ein und lässt den aufgebauten Unterdruck wieder zusammenfallen. Die Druckverhältnisse zwischen den Pleurablättern sind auch wegen der natürlichen Atembewegung des Patienten nicht stabil und erschweren das Ausheilen der verletzten Pleura. Der erforderliche Unterdruck bei Pneumathoraxbehandlungen wird deshalb bis heute vorzugsweise mit kommunizierenden Druckausgleichsflaschen erzeugt.

Es ist deshalb Aufgabe der vorliegenden Erfindung eine elektrisch betriebene, kostengünstige und betriebssichere medizinPumpe zu schaffen, welche die Nachteile der bekannten Vorrichtungen überwindet und insbesondere die individuelle Einstellung des zeitlichen Druckverlaufs, bspw. der Saugkraft und des Saugrhythmus erlaubt und insbesondere unabhängig vom genauen Sitz der Saugglocke sicher arbeitet.

Diese Aufgabe wird bei einer medizintechnischen Pumpe der eingangs genannten Art erfindungsgemäss durch die kennzeichnenden Merkmale des Patentanspruchs 1 gelöst. Weitere Ausbildungen und besondere Ausführungsformen der erfindungsgemässen Pumpe sind in den abhängigen Patentansprüchen definiert.

Insbesondere weist die erfindungsgemässe Pumpe einen Pumpenantrieb auf, dessen Bewegungsablauf nicht kontinuierlich ist. Erfindungsgemäss wird ein Antrieb verwendet, dessen Bewegungsrichtung mit einer einstellbaren Frequenz wechselt.
Zur Regelung der mechanischen Bewegung des Antriebs ist eine elektronische Schaltung vorgesehen, mit welcher die Stromstärke und insbesondere die Stromrichtung resp. die Phasenverschiebung des Antriebsstroms geregelt werden kann. Diese Änderungen des Antriebsstroms sollen im Folgenden unter dem Begriff "Umformung" erfasst sein. Da die am Pumpzylinder benötigte Kraft proportional zum erzeugten Unterdruck ist und die aufgebrachte Kraft wiederum proportional zum Antriebsstrom ist, lässt sich durch die Regulierung des Antriebsstrom in einfacher Weise eine gewünschte Unterdruckbegrenzung erzielen resp. der zeitliche Verlauf des erzeugten Unterdrucks in gewünschter Weise regeln.

Die erfindungsgemässe elektronische Regelschaltung weist also Mittel auf, um den vom Pumpenantrieb benötigten Strom zu messen. Dieser gemessene Wert wird einem Motorstrombegrenzer zugeführt und wird mit dem Sollwert eines Stellwertgebers für die Begrenzung des Maximalunterdrucks verglichen. Sobald der gemessene Wert den Sollwert überschreitet wird der Strom für den Pumpenantrieb auf diesen Sollwert begrenzt.

Es versteht sich, dass für den Pumpenantrieb Elektromagnete, Linearversteller, Gleich- oder Wechselstrommotoren u. a. verwendet werden können. Die mit diesem Antrieb zu erzeugende Pumpbewegung kann ohne weiteres erfinderisches Tun mit einem Hebelsystem, mit einer Spindelanordnung oder ähnlichem bewirkt werden. Je nach verwendetem Antrieb ist es angezeigt, die momentane Position des Antriebelementes zu erfassen, insbesondere auch um die Hublänge der Pumpe definieren resp. einstellen zu können. Ebenso sieht die erfindungsgemässe Schaltung vor, den Taktgeber einstellbar zu gestalten. Damit kann der zeitliche Verlauf des Unterdrucks und die Mengenleistung der angesaugten Luft elektronisch gesteuert und damit jede gewünschte Saugkurve erzeugt werden. Zudem können verfälschende Eigenschaften des Antriebs, wie bspw. Reibung und Hysterese korrigiert und kompensiert werden. Grundsätzlich lässt sich der Unterdruck durch eine Kraftmessung mit geeigneten Sensoren wie bspw. Biegebalken mit Dehnmessstreifen ermitteln und im Regelkreis verarbeiten.

Im folgenden soll die Erfindung an Hand von bevorzugten Ausführungsbeispielen und mit Hilfe der Zeichnungen näher erläutert werden. Es zeigt:
- Figur 1:: ein Prinzipschaltbild der erfindungsgemässen elektronischen Regelschaltung;
- Figuren 2a und 2b:: bevorzugte Ausführungsformen für den Antrieb der Pumpe;
- Figur 3:: ein detailliertes Schaltbild der erfindungsgemässen elektronischen Regelung.

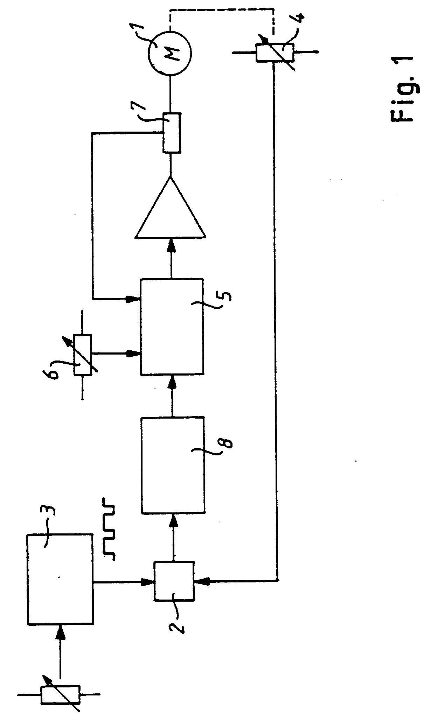

Das in Figur 1 dargestellte Prinzipschaltbild zeigt die erfindungsgemässe elektronische Regelschaltung für den Antrieb 1 der Pumpzylinderanordnung. Dieser Antrieb ist beispielsweise als Elektromotor oder als Elektromagnet ausgebildet. Der Stromversorgungsteil für diesen Antrieb 1 weist erfindungsgemäss einen Soll-Istwert-Vergleicher 2 auf. In einer bevorzugten Ausführungsform wird dieses Bauteil 2 von einem regelbaren Taktgeber 3 gesteuert wird. Es versteht sich, dass der Schaltzeitpunkt für die Umformung mit der gewünschten Stellung der Pumpzylinderanordnung abstimmbar ist. Diese Abstimmung kann fest eingestellt sein, ist jedoch in einer bevorzugten Ausführungsform durch ein regelbares Positionserfassungsmittel 4 realisiert. Mit diesem Positionserfassungsmittel 4 wird das Saugvolumen der Pumpzylinderanordnung erfasst und mit Hilfe eines von diesen Mitteln 4 erzeugten Signals begrenzt. Insbesondere wird dieses Signal zum periodischen Umformen des Antriebsstroms oder zu dessen Phasenverschiebung den Mitteln 2, 8 und 5 zugeführt.

Um die Saugkraft der Pumpe zu regeln, ist eine regelbare Strombegrenzungsvorrichtung 5 vorgesehen. Diese Strombegrenzungsvorrichtung 5 umfasst einen Stellwertgeber 6, dessen Signal erfindungsgemäss mit einem von Mitteln 7 zum Messen des Antriebsstroms erzeugten Ist-Wertsignals verglichen wird. Der gemessene Ist-Wert des Antriebstroms ist direkt proportional zum Moment des Motors und damit auch zur Saugkraft.

In einer Weiterbildung der erfindungsgemässen Brustpumpe umfasst die Pumpzylinderanordnug mehrere Pumpzylinder, welche zur Erzeugung eines gleichbleibenden Saugdrucks von einer Positionsregelschaltung 8 in geeigneter Weise angesteuert werden. Insbesondere sind Mittel vorgesehen, welche die Position der einzelnen Pumpzylinder erfassen. Erreicht einer der Pumpzylinder eine vorgegebene Endposition, setzt die Positionsregelschaltung 8 den nächsten Pumpzylinder in Betrieb.

Diese Positionsregelschaltung 8 definiert auch elektronisch den Umkehrpunkt und insbesondere die Nullpunktstellung für den jeweiligen Pumpzylinder. Dies ist von besonderer Bedeutung, da sich das Pumpvolumen bspw. bei einer Brustpumpe während des Pumpens verändern kann und damit ein hysteresisartiger Verlauf des Saugdrucks entstehen kann. Durch das Abtasten des Ist-Wertes des Antriebsstroms kann damit unabhängig von der mechanischen Nullpunktstellung der tatsächlich anliegende Unterdruck respektive das Vorliegen von Normaldruck im Pumpzylinder festgestellt werden.
Bei der Verwendung von mehreren Pumpzylindern kann damit auch ein vorbestimmter Haltedruck über einen längeren Zeitraum erzeugt werden, was inbesondere bei Toraxoperationen von Bedeutung ist. Insbesondere erlaubt die vorliegende Positionsregelschaltung 8 das Aufrechterhalten des gewünschten Unterdruck trotz Versagen eines Pumpzylinders oder beim Lecken eines Unterdrucksystems und weist damit in hohem Masse eine für medizintechnische Pumpen notwendige Funktionssicherheit auf. Ebenso kann mit der erfindungsgemässen Pumpe die obere Grenze für den maximal zu erzeugenden Saugdruck festgelegt werden. In einer Weiterbildung der erfindungsgemässen Pumpe wird diese obere Stromgrenze während eines vorbestimmten Zeitintervalls rhythmisch variiert um bspw. die Milchproduktion zu stimulieren.

Die erfindungsgemässe Pumpe erlaubt somit erstmals mit einfachen kostengünstigen Mitteln die Erzeugung eines individuell einstellbaren Pumpverhaltens, d.h. die freie Wahl des Pumpvolumens, der Pumpzeit, des Pumprhythmus sowie der Pumpkraft und deren zeitlichen Verlauf.

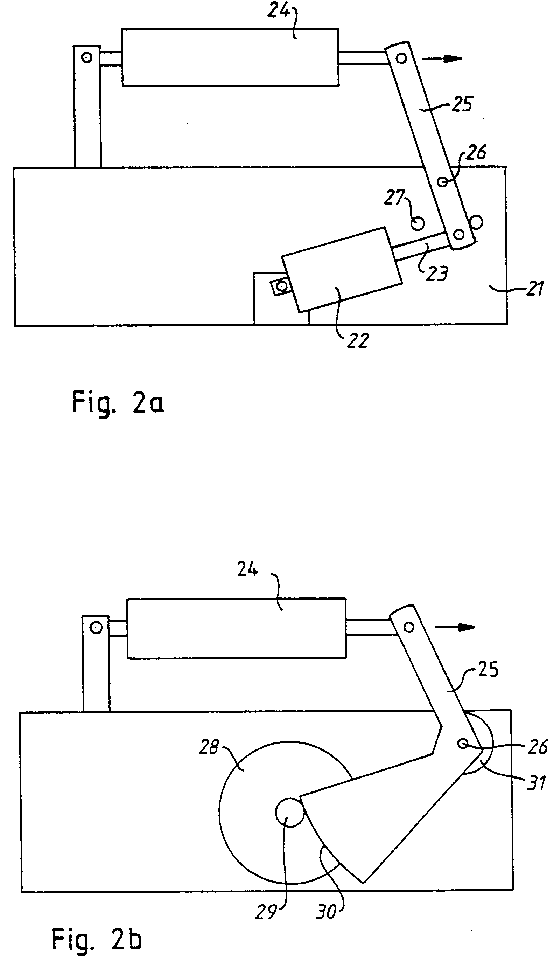

Die Figuren 2a und 2b stellen den mechanischen Aufbau zweier Ausführungsformen der erfindungsgemässen Pumpe dar. Dabei zeigt Figur 2a ein Gehäuseteil 21, in welchem Gehäuse mindestens ein Elektromagnet 22 angebracht ist. Der Kern 23 dieses Elektromaggneten 22 ist mit einem an einer Pumpzylinderanordnung 24 angelenkten Hebel 25 verbunden, welcher Hebel 25 um einen gehäusefesten Zapfen 26 drehbar ist. Ein Anschlagstift 27, dessen Position mechanisch oder elektrisch fixierbar ist, begrenzt das Pumpvolumen in einfacher Weise. Die gewünschte Saugkraft ist direkt proportional zum einstellbaren Antriebsstroms des Elektromagneten 22.

Die in Figur 2b dargestellte Ausführungsform weist einen Elektromotor 28 mit einer als Drehmomentbegrenzung wirkender Strombegrenzung auf und wirkt mittels einer geeigneten Verzahnung 29, 30 mit dem Hebel 25 zusammen. Dieser Hebel 25 ist um einen gehäusefesten Zapfen 26 drehbar gelagert und treibt die Pumpzylinderanordnung 24. Ein am gehäusefesten Zapfen 26 angebrachtes Potentiometer 31 dient der Erfassung der Hebelposition und damit des Pumpvolumens.

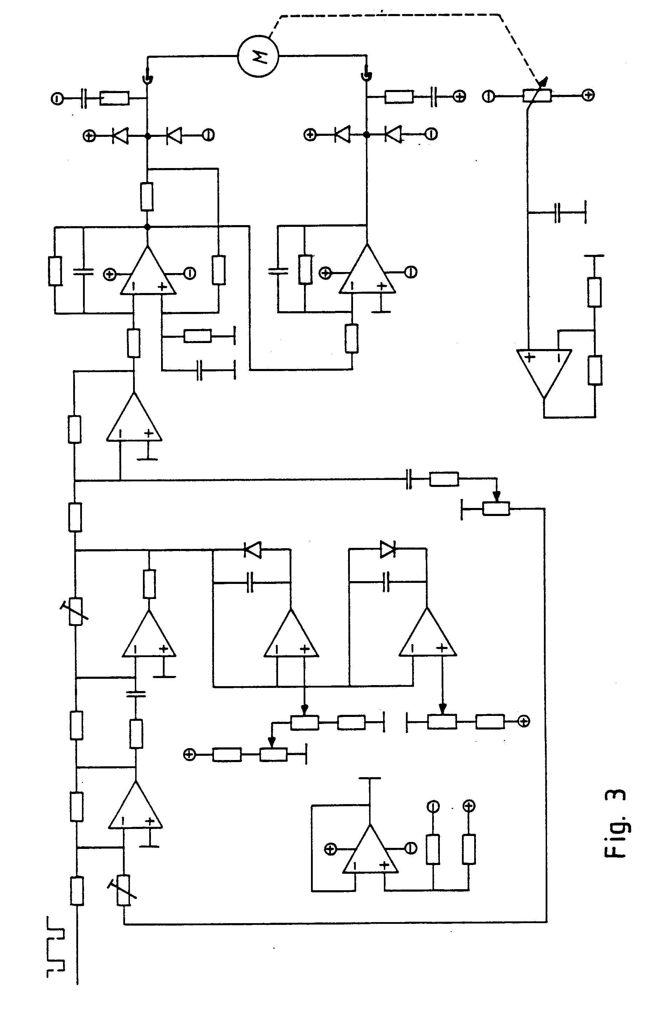

Das in Figur 3 dargestellte Schaltbild zeigt den elektronischen Aufbau einer erfindungsgemässen Analogregelung im Detail und ist für den Fachmann unmittelbar nachvollziehbar, weshalb diese hier nicht mehr erläutert zu werden braucht.

Die erfindungsgemässe Regelung kann als gedruckte Analogschaltung ebenso gut realisiert sein wie als digitale Mikroprozessorschaltung.

## Patentansprüche

1. Medizintechnische Pumpe mit mindestens einer Pumpzylinderanordnung (24), einem elektrischen Antrieb (1) dafür, einem Stromversorgungsteil zur Erzeugung eines Antriebsstroms für diesen Antrieb (1) und einer regelbaren Strombegrenzungsvorrichtung (5), dadurch gekennzeichnet, dass Mittel (2,8,5) vorgesehen sind, mit welchen der Antriebsstrom geregelt wird und periodisch umgeformt wird, wobei diese Mittel eine gesteuerte Stromwertgeberschaltung (2,5,8) umfassen.

2. Pumpe nach Anspruch 1, dadurch gekennzeichnet, dass die Mittel zum periodischen Umformen des Antriebsstroms einen regelbaren Taktgeber (3) umfassen.

3. Pumpe nach Anspruch 2, dadurch gekennzeichnet, dass Mittel (7) vorgesehen sind, mit welchen der Antriebsstrom gemessen wird.

4. Pumpe nach Anspruch 3, dadurch gekennzeichnet, dass die Mittel (7) zum Messen des Antriebsstroms mit einer einstellbaren Strombegrenzungsvorrichtung (5) für den Antrieb (1) verbunden sind, um ein von diesen Mitteln (7) erzeugtes elektrisches Signal als Ist-Wert dieser Strombegrenzungsvorrichtung (5) zuführen zu können.

5. Pumpe nach Anspruch 4, dadurch gekennzeichnet, dass Mittel (4) zur Erfassung der mechanischen Position der Pumpzylinderanordnung vorgesehen sind, welche ein elektrisches Signal erzeugen und welche mit den Mitteln (2,5,8) zum periodischen Umformen des Antriebsstroms verbunden sind.

6. Pumpe nach Anspruch 4, dadurch gekennzeichnet, dass diese eine Positionsregelschaltung (8) aufweist, mit welcher mehrere Pumpzylinder in vorgegebener Weise ansteuerbar sind.

7. Pumpe nach Anspruch 1, dadurch gekennzeichnet, dass diese zur Regelung des Antriebsstroms einen Mikroprozessor aufweist.

8. Pumpe nach Anspruch 1, dadurch gekennzeichnet, dass diese als Brustpumpe ausgebildet ist.

9. Pumpe nach Anspruch 1, dadurch gekennzeichnet, dass diese als Thoraxpumpe ausgebildet ist.

## Claims

1. Pump for use in medical technology comprising at least one pump cylinder unit (24), an electrical drive (1) for said unit, a current supply unit for generating a driving current for said electrical drive (1) and an adjustable current-limiting device (5), characterized in that means (2, 8, 5) are provided to regulate and to periodically transform the driving current, said means including a controlled current set-point adjuster (2, 5, 8).

2. Pump according to claim 1, characterized in that said means for periodically transforming the driving current include an adjustable clock generator (3).

3. Pump according to claim 2, characterized in that means (7) are provided for measuring the driving current.

4. Pump according to claim 3, characterized in that the means (7) for measuring the driving current are connected to said adjustable current limiting device (5) for the electrical drive (1), so as to be able to feed an electrical signal that is generated by said means (7) as an actual value to said current limiting device (5).

5. Pump according to claim 4, characterized in that means (4) for detecting a mechanical position of the pump cylinder unit are provided, said means (4) generating an electrical signal and being connected to the means (2, 5, 8) for periodically transforming the driving current.

6. Pump according to claim 4, characterized in that said pump comprises a position control circuit (8), by means of which several pump cylinders can be triggered in a predetermined manner.

7. Pump according to claim 1, characterized in that said pump comprises a microprocessor for controlling the driving current.

8. Pump according to claim 1, characterized in that said pump is designed as a breast pump.

9. Pump according to claim 1, characterized in that said pump is designed as a thorax pump.

## Revendications

1. Pompe médicale comprenant au moins un dispositif de pompage à corps cylindrique (24), un entraînement électrique (1) pour ce dispositif, une alimentation électrique fournissant un courant d'alimentation à cet entraînement (1) et d'un dispositif réglable limiteur de courant (5), caractérisée en ce que des moyens (2, 8, 5) sont prévus avec lesquels le courant d'alimentation est régulé et est périodiquement converti, ces moyens comprenant un circuit d'asservissement (2, 5, 8) du courant de commande.

2. Pompe selon la revendication 1, caractérisée en ce que les moyens de conversion périodique du courant d'alimentation comprennent un générateur d'impulsions (3) réglable.

3. Pompe selon la revendication 2, caractérisée en ce que des moyens (7) sont prévus avec lesquels le courant d'alimentation est mesuré.

4. Pompe selon la revendication 3, caractérisée en ce que les moyens (7) de mesure du courant d'alimentation sont reliés à un dispositif limiteur de courant réglable (5) pour l'entraînement (1), afin de pouvoir fournir le signal électrique délivré par ces moyens (7) en tant que valeur instantanée à ce dispositif limiteur de courant (5).

5. Pompe selon la revendication 4, caractérisée en ce que des moyens (4) de détermination de la position mécanique du dispositif de pompage à corps cylindrique sont prévus, et que ces moyens (4) fournissent un signal électrique et sont reliés aux moyens (3, 5, 8) de conversion périodique du courant d'alimentation.

6. Pompe selon la revendication 4, caractérisée en ce que celle-ci comprend un circuit d'asservissement en position (8) avec lequel plusieurs pompes à piston cylindrique peuvent être commandées de façon prédéterminée.

7. Pompe selon la revendication 1, caractérisée en ce que celle-ci comprend un microprocesseur pour la régulation du courant d'alimentation.

8. Pompe selon la revendication 1, caractérisée en ce que celle-ci est réalisée sous forme de tire-lait.

9. Pompe selon la revendication 1, caractérisée en ce que celle-ci est réalisée sous forme de pompe thoracique.
